# EUROPEAN PATENT SPECIFICATION

(11) **EP 4 504 836 B1**
(45) Date of publication and mention of the grant of the patent: **13.08.2025**
(21) Application number: 24716424.7
(22) Date of filing: 11.04.2024
(51) Int. Cl.: C08L 89/06, C07K 1/14, C07K 14/78, A61K 38/01

(54) **THE USE OF FATTY ALCOHOL POLYGLYCOL ETHER TO REDUCE ENDOTOXIN ACTIVITY AND/OR ENDOTOXINS IN COLLAGEN-CONTAINING AND/OR COLLAGEN-DERIVED PRODUCTS**
VERWENDUNG VON FETTALKOHOLPOLYGLYKOLETHER ZUR REDUZIERUNG DER ENDOTOXINAKTIVITÄT UND/ODER ENDOTOXINE IN KOLLAGENHALTIGEN UND/ODER KOLLAGENABGELEITETEN PRODUKTEN
UTILISATION D'ÉTHER DE POLYGLYCOL D'ALCOOL GRAS POUR RÉDUIRE L'ACTIVITÉ D'ENDOTOXINE ET/OU LES ENDOTOXINES DANS DES PRODUITS CONTENANT DU COLLAGÈNE ET/OU DÉRIVÉS DE COLLAGÈNE

(30) Priority: 11.04.2023 BE 202305269
(43) Date of publication of application: 12.02.2025
(73) Proprietor: Rousselot BV, 9000 Gent (BE)
(72) Inventor: VAN DER MEEREN, Ruben Jean Antoinette, 9000 GENT (BE); ASDI BASKORO SEJATI, Daniel, 9000 GENT (BE)
(74) Representative: EP&C
(86) International application number: PCT/EP2024/059850
(87) International publication number: WO 2024/213643

(56) References cited:
- WO-A1-2016/085345
- BHAIRI SRIRAMA M.: "CALBIOCHEM DETERGENTS A guide to the properties and uses of detergents in biological systems", 1 January 2001 (2001-01-01), XP055926329, Retrieved from the Internet <URL:http://wolfson.huji.ac.il/purification/PDF/detergents/calbiochem_detergents.pdf> [retrieved on 20220531]
- KLAMMT CHRISTIAN ET AL: "Evaluation of detergents for the soluble expression of alpha-helical and beta-barrel-type integral membrane proteins by a preparative scale individual cell-free expression system", THE FEBS JOURNAL, WILEY-BLACKWELL PUBLISHING LTD, GB, vol. 272, no. 23, 1 December 2005 (2005-12-01), pages 6024 - 6038, XP002460325, ISSN: 1742-464X, DOI: 10.1111/J.1742-4658.2005.05002.X

## Description

### TECHNICAL FIELD

The present invention relates to reducing lipopolysaccharides (LPS) activity and/or LPS in collagen-containing and/or collagen-derived products and products obtained by the method.

### BACKGROUND OF THE INVENTION

Collagen, collagen-containing or collagen-derived products, such as extracellular matrix (ECM), collagen peptides, gelatin and (chemically) modified gelatins have found applications in a variety of different areas, ranging from the food industry to the medical and pharmaceutical field. The interest in these type of materials stems from their desirable features, which include their biocompatibility and biodegradability. Even though collagen and collagen-containing and collagen-derived materials are generally considered safe, their biomedical use is challenged by the presence of endotoxins, such as LPS.

LPS are localized on the outer cell membrane of gram-negative bacteria where they provide structural integrity and protection by acting as a permeability barrier. LPS consist of a variable polysaccharide chain and a lipid moiety, lipid A. LPS molecules are about 10 kDa in size, but can form large aggregates in aqueous media, also named 'micelles'. LPS are not secreted by bacteria, but released upon disruption of the bacterial cell membrane, hence the name 'endotoxins'. LPS are highly immunogenic to humans and even exposure to low amounts of LPS can have drastic adverse effects. Because of the toxicity, the amount of endotoxins permitted on medical devices is strictly regulated. For example, the Food and Drug Administration (FDA) allows a maximum of 0.5 endotoxin unit (EU)/g or 20 EU/device for products that (in)directly contact the cardiovascular and lymphatic system. For devices in contact with cerebrospinal fluid, this limit is even lower.

In traditional manufacturing processes of collagen-containing and/or collagen-derived products, it is challenging to avoid endotoxin contamination. Moreover, once endotoxins are present, these are extremely difficult to remove, especially from sensitive substances comprising proteins.

In the art, methods for removing LPS from protein solutions using surfactants have been described, for example in WO2016085345. While showing great promise, the aforementioned approach relies on Triton X-100, of which its use is under discussion.

Thus, there remains an unmet need to reduce and/or remove LPS from collagen-containing and collagen-derived products, in an efficient and safe manner.

It is an objective of the present invention to overcome one or more of the above-mentioned problems. To this end, the present invention provides a method for reducing LPS activity and/or LPS from collagen-containing and/or collagen-derived products.

### SUMMARY OF THE INVENTION

The present inventors identified an effective method to reduce LPS activity and/or LPS in collagen-containing and/or collagen-derived products, which is based on the use of fatty alcohol polyglycol ethers.

More specifically, the present inventors found that Laureth-9, which is a non-ionic, non-toxic micelle forming surfactant, is effective in reducing LPS activity in collagen-containing and/or collagen-derived products. Unexpectedly, it was found that the concentration of Laureth-9 required to be effective is significantly lower than for Triton X-100.

The present inventors thus offer an improved method to reduce LPS activity.

In one aspect, the present invention relates to a method for reducing LPS activity in a collagen-containing and/or a collagen-derived product.

In one aspect, the present invention relates to the use of a polyglycol ether of a fatty alcohol to reduce LPS activity in a collagen-containing and/or a collagen-derived product.

In one aspect, the present invention relates to a collagen-containing and/or collagen-derived product, having an LPS content of less than 3000 EU/g, preferably less than 1000 EU/g, more preferably less than 100 EU/g, even more preferably less than 10 EU/g, most preferably less than 1 EU/g.

### DETAILED DESCRIPTION OF THE INVENTION

The present invention relates to a method for reducing LPS activity in a collagen-containing and/or a collagen-derived product, said method comprising one or more steps of, preferably all steps of:
- providing a collagen-containing and/or collagen-derived product comprising LPS,
- contacting the collagen-containing and/or collagen-derived product comprising LPS with a polyglycol ether of a fatty alcohol to provide a mixture,
- recovering a collagen-containing and/or collagen-derived product, preferably a collagen-containing and/or collagen-derived product wherein the LPS content is less than 3000 EU/g, more preferably wherein the LPS content is less than 1000 EU/g, even more preferably wherein the LPS content is less than 100 EU/g, yet even more preferably wherein the LPS content is less than 10 EU/g, most preferably wherein the LPS content is less than 1 EU/g.

The term 'reducing LPS activity' as used herein means that the LPS level of the recovered collagen-containing and/or collagen-derived product is lower than that of the starting material, i.e. the collagen-containing and/or collagen-derived product before being contacted with a polyglycol ether of a fatty alcohol. In the present invention, 'reducing LPS activity' comprises both a reduction and/or removal of LPS in and/or from the starting material. This reduction and/or removal of LPS in and/or from the starting material can be determined using the Limulus Amebocyte Lysate (LAL) assay, which is a well-known method for the detection of LPS. LAL is an aqueous extract of blood cells (amebocytes) from the horseshoe crab Limulus polyphemus. The reaction between LAL and LPS is the basis of the LAL assay and is used to qualitatively (e.g. visual inspection) or quantitatively (e.g. chromogenic or turbidimetric) determine LPS content.

In an embodiment, the LPS activity of the recovered collagen-containing and/or collagen-derived product is preferably at least 10% lower than the LPS activity of the starting material. More preferably, the LPS activity of the recovered collagen-containing and/or collagen-derived product is at least 20%, 30%, 40%, 50%, 60%, 70%, 80% lower than the LPS activity of the starting material. Preferably, the starting material has an LPS activity of less than 20000 EU/g per gram dry weight of collagen-containing and/or collagen-derived product. More preferably, the starting material has an LPS activity of less than 15000 EU/g per gram dry weight of collagen-containing and/or collagen-derived product, even more preferably less than 5000 EU/g per gram dry weight of collagen-containing an/or collagen-derived product. The starting material may also have a higher or lower LPS activity, and if the LPS content is higher, the material may be pre-treated e.g. with an ion exchange chromatography step.

In an embodiment, the collagen-containing and/or collagen-derived product is endotoxin-free (i.e LPS free). The term "endotoxin-free" can mean absence of endotoxin and/or can mean "essentially free of endotoxin" (i.e. essentially free of LPS).

The term "free of" can mean the same as "essentially free of". The term "essentially free" encompasses that the amount of a substance or compound is not measurable according to a standard technique in the field and/or is below a certain threshold. In addition or alternatively, the term "essentially free" may mean that the endotoxin level is below 10, preferably below 1, more preferably below 0.1, even more preferably below 0.01, most preferably below 0.001 (all in EU/g or EU/ml). The wordings "free" or "essentially free" encompass that a substance or compound is completely absent (e.g. 0 wt.% or 0 EU/g). The terms "in absence", "free" and "essentially free" can be used interchangeably in the context of the current invention.

It is particularly advantageous to use the polyglycol ether of a fatty alcohol for removal of LPS (activity) as described herein, if the starting material has a high amount of LPS (activity). For example, it was found that the polyglycol ether of a fatty alcohol is more effective in removing LPS (activity) than other known methods such as Triton X-100 if the LPS content in the starting material is relatively high, such as at least 1000 EU/g, preferably at least 2500 EU/g, more preferably at least 5000 EU/g, even more preferably at least 10000 EU/g, most preferably at least 50000 EU/g. In an embodiment, the LPS content in the collagen-containing and/or collagen-derived product is at least 500 EU/g, or at least 1000 EU/g, or at least 2500 EU/g, or at least 5000 EU/g, or at least 10000 EU/g, or at least at least 50000 EU/g and/or no more than 50000 EU/g, or no more than 10000 EU/g, or no more than 5000 EU/g, or no more than 2500 EU/g, or no more than 1000 EU/g or no more than 500 EU/g. In addition or alternatively, the LPS content in the collagen-containing and/or collagen-derived product is preferably between 500 - 50000 EU/g, 1000 - 50000 EU/g, most preferably 1000 - 10000 EU/g.

The present disclosure comprises contacting a collagen-containing and/or collagen-derived product with a polyglycol ether of a fatty alcohol. The purpose can be to provide a mixture or a blend such that an interaction between the collagen-containing and/or collagen-derived product and polyglycol ether of a fatty alcohol is provided.

In an embodiment, the collagen-containing and/or collagen-derived product can be extracellular matrix, native collagen, gelatin, (chemically) modified gelatin, gelatin hydrolysate, and mixtures thereof. Native collagen may comprise acid-extracted and enzyme soluble collagen, including telocollagen, atelocollagen and fibrillar collagen.

The term "collagen" in the context of the current invention means an amino acid sequence comprising a repeating (Gly-X-Y) sequence, preferably comprising at least 2, 3, 4, 5, 10, 20, 50, 100, 200, 300, or 400 sequences containing the sequence Gly-X-Y, where X and Y are an amino acid residue independently chosen from each other, but X and/or Y are more preferably proline. The "collagen" preferably has a sequence found in native collagen in one or more animal species. In addition or alternatively, the "collagen" can mean a full-length sequence or fragment or subunit thereof of (native) collagen, preferably one or more of collagen types I - XXVII, more preferably one or even more of type I, II, III, V, or X collagen, even more preferably one or more of type I, II or III collagen. For example, the term "collagen" may refer to an alpha-1(I), alpha-2(I), alpha-1(ll) or alpha-1(lll) chain, or a fragment thereof. The term "collagen" encompasses the triple helix structure as formed by three subunits as present in native collagen.

The "collagen" in the context of the current invention encompasses "gelatin", "collagen hydrolysate" and "hydrolysed gelatin".

The term "collagen hydrolysate" the context of the current invention means a mix of short chains of amino acids (di-, tri, oligopeptides, polypeptides) derived from (partial) hydrolysis from native (full-length) collagen, such as by enzymatic hydrolysis. The degree of hydrolysis has an impact on the average molecular weight (expressed in Dalton, Da) of the final product. The term "collagen hydrolysate" may be used interchangeably and synonymous with the terms "hydrolysed collagen" or "collagen peptide". The "collagen hydrolysate" in the context of the current invention encompasses collagen which is subjected to hydrolysis or partial hydrolysis. The "collagen hydrolysate" in the context of the current invention may be produced from a collagen-containing material in a one-step process or via an intermediate gelatin stage (i.e. thus "hydrolysed gelatin" is obtained). The term "collagen hydrolysate" thus encompasses hydrolysed gelatin (i.e. hydrolyzed gelatin).

The collagen-containing and/or collagen-derived products as used herein may be derived from one or more types of collagen known in the art. More specifically, the collagen-containing and/or collagen-derived products as used herein may be selected from the group comprising collagen type I, II and III, IV, V, VI, VII, VIII, IX, X, XI, XII, XIII, XIV, XV, XVI, XVII, XVIII, XIX, XX, XXI, XXII, XXIII, XXIV, XXV, XXVI, XXVII, XXVIII. In addition or alternatively, the collagen-containing and/or collagen-derived products as used herein may be a mixture of one or more types of collagen.

The collagen-containing and/or collagen-derived products may be from synthetic nature (i.e. recombinant collagen), but are preferably derived from any animal raw material from which collagen-containing and/or collagen-derived products can be prepared. In a preferred embodiment, the animal raw material is from bovine, porcine, poultry or fish origin. In another preferred embodiment, the collagen-containing and/or collagen-derived product is derived from skin and/or skin connective tissue. In yet another preferred embodiment, the collagen-containing and/or collagen-derived product is derived from cartilage. In yet another preferred embodiment, the collagen-containing and/or collagen-derived product is derived from bone.

The term 'collagen-derived product' as used herein refers to proteins and peptides that were part of the collagen matrix in the raw material and were processed to prepare the collagen-derived products, e.g. gelatin, as known in the art. The gelatin and/or (chemically) modified gelatins may be produced by (partial) alkaline and/or acid hydrolysis, by enzymatic hydrolysis, by thermal hydrolysis and/or a combination thereof. Gelatin does not constitute a uniform protein molecule, but comprises a variable amount of protein molecules of variable length. Preferably, the gelatin used herein has an average molecular weight within the range of 1500 Da to 300 kDa, preferably between 2000 Da and 300 kDa, between 4000 Da and 300 kDa, between 5000 Da and 300 kDa, between 10 kDa and 300 kDa, or between 20 kDa and 300 kDa, more preferably between 50 kDa and 300 kDa, most preferably between 100 kDa and 300 kDa, such as between 100 kDa and 275 kDa or between 100 kDa and 250 kDa. The term '(chemically) modified gelatins' comprises gelatins with one or more functionalized groups to the gelatin backbone, such as acrylamides, ferulic acid, methacryloyl (e.g. GeIMA), desaminotyrosine (e.g. GeIDAT), furfurylamine and norbornene (Klotz, B. J., Gawlitta, D., Rosenberg, A. J., Malda, J., & Melchels, F. P. (2016). Gelatin-methacryloyl hydrogels: towards biofabrication-based tissue repair. Trends in biotechnology, 34(5), 394-407). 'Collagen-derived' products also include products that may be obtained after further processing of gelatin, such as gelatin hydrolysate, which is a peptide preparation originating from hydrolysis of gelatin to peptide molecules having an average molecular weight of 70 kDa or less, usually 20 kDA or less, usually between 100 and 15000 Da.

In an embodiment, the collagen-containing and/or collagen-derived product can be in the form of a solution, wherein the solvent may comprise an acid (e.g. acetic acid, formic acid) or an organic solvent (e.g. supercritical carbon dioxide, acetone, preferably up to 30%). The collagen-containing and/or collagen-derived product can also be in the form of an aqueous solution, which may comprise any type of water such as tap water, distilled water, deionized water and/or ultra-pure water. The term "aqueous solution" in the context of the current invention encompass solutions comprising in addition to water further one or more co-solvents, which may depend on the polyglycol ether of a fatty alcohol of choice. The presence of the co-solvent may for example improve the solubility of the polyglycol ether of a fatty alcohol. Examples of co-solvents or surfactants the skilled person is aware of and suitable for the present invention is one or more selected from the group consisting of ethanol, isopropyl alcohol, propylene glycol, polyethylene glycol (PEG), sodium lauryl sulfate, polysorbate surfactants (e.g., Polysorbate 20, Polysorbate 80, sorbitan esters (e.g. sorbitan monolaurate, sorbitan monooleate), ethoxylated alcohols, ethoxylated fatty acids, ethoxylated fatty amines, preferably ethanol.

The solution can be prepared by dissolving (dry) collagen-containing and/or collagen-derived product with the solvent. The collagen-containing and/or collagen-derived product is preferably dissolved at room temperature or elevated temperature, however preferably not above 68 °C, more preferably not above 65 °C, even more preferably not above 60 °C, as elevated temperatures may increase undesired hydrolysis of the collagen-containing and/or collagen-derived product. It is possible to perform the previous steps at different temperatures, however preferably with a maximum of 68 °C. Preferably, the mixing time comprises a period of at least 10 minutes. It is also possible to use a shorter or longer mixing time, however, for effectiveness and efficiency, the mixing time is preferably between 10 to 60 minutes. Alternatively, the collagen-containing and/or collagen-derived product can be contacted with a pure solution of Laureth-9 directly.

In an embodiment, the aqueous solution may comprise any concentration of collagen-containing and/or collagen-derived product. In a preferred embodiment, the aqueous solution comprises at least 1 w/w% collagen-containing and/or collagen-derived product, preferably at least 4 w/w%, more preferably at least 6 w/w%, even more preferably at least 8 w/w% collagen-containing and/or collagen-derived product, yet even more preferably at least 10 w/w% collagen-containing and/or collagen-derived product, yet even more preferably at least 15 w/w% collagen-containing and/or collagen-derived product, most preferably at least 20 w/w%.

In another preferred embodiment, the aqueous solution comprises between 1-50 w/w% collagen-containing and/or collagen-derived product, preferably between 2-30 w/w%, more preferably between 4-15 w/w% collagen-containing and/or collagen-derived product, even more preferably between 6-10 w/w% collagen-containing and/or collagen-derived product. The aqueous medium may comprise as much collagen-containing and/or collagen-derived product, up to until the aqueous solution becomes too viscous for processing as according to the method. The aqueous solution of the present disclosure preferably comprises at most 55, 50, 45, 40, 35, 30, 25, 20, 15, 10 w/w% collagen-containing and/or collagen-derived product. Higher concentrations of collagen-containing and/or collagen-derived product, such as at least 60 w/w%, 70 w/w%, 80 w/w% and 90 w/w% may be possible when a collagen-containing and/or collagen-derived product with a relatively low molecular weight is used, such as gelatin hydrolysate. Whereas it may be possible to reduce the viscosity of the collagen-containing and/or collagen-derived aqueous solution by increasing the temperature, elevated temperatures may result in undesired hydrolysis of the collagen-containing and/or collagen-derived product.

The polyglycol ether of a fatty alcohol can be a polyglycol ether of a C8-C20 fatty alcohol, preferably of a C8-C18 fatty alcohol, more preferably of a C8-C16 fatty alcohol, most preferably of a C10-C14 fatty alcohol. In a preferred embodiment, the polyglycol ether of a fatty alcohol is preferably selected from the list comprising a polyglycol ether of 1-decanol, 1-dodecanol, 1-tetradecanol, 1-cetylacohol, 1-palmitoleyl alcohol, 1-octadecenol alcohol and/or 1-stearyl alcohol. Most preferably, the polyglycol ether of a fatty alcohol is a polyglycol ether of 1-dodecanol.

In the present disclosure, fatty alcohols (or long-chain alcohols) are typically straight-chain primary alcohols, and can be typically derived from natural fats and oils. Some relevant fatty alcohols are lauryl (C12), myristyl (C14), stearyl (C18), and oleyl (C16) alcohols. Fatty alcohols typically have an even number of carbon atoms and a single alcohol group (-OH) attached to the terminal carbon. Some are unsaturated and some are branched. As with fatty acids, they are often referred to generically by the number of carbon atoms in the molecule, such as "a C12 alcohol", that is an alcohol having 12 carbons, for example dodecanol.

In an embodiment, the polyglycol ether of a fatty alcohol contains and/or contains on average between 1-20 oxyethylene groups, preferably between 5-18 oxyethylene groups, more preferably between 8-16 oxyethylene groups, most preferably between 10-14 oxyethylene groups.

In a more preferred embodiment, the polyglycol ether of a fatty alcohol contains and/or contains on average between 1-100 oxyethylene groups, more preferably between 2-75 oxyethylene groups, even more preferably between 3-50 oxyethylene groups, yet even more preferably 4-40 oxyethylene groups, yet even more preferably 5-30 oxyethylene groups, yet even more preferably 6-20 oxyethylene groups, most preferably between 7-10 oxyethylene groups. In another preferred embodiment, the polyglycol ether of a fatty alcohol contains and/or contains on average at least 1, 2, 3, 4, 5, 6, 7, 8, 9 oxyethylene groups. In yet another preferred embodiment, the polyglycol ether of a fatty alcohol contains and/or contains on average at most 200, 175, 150, 125, 100, 75, 50, 45, 40, 39, 38, 37, 36, 35, 34, 33, 32, 31, 30, 29, 28, 27, 26, 25, 24, 23, 22, 21, 20, 19, 18, 17, 16, 15, 14, 13, 12, 11, 10, 9 oxyethylene groups. In yet another preferred embodiment, the polyglycol ether of a fatty alcohol is preferably selected from one or more polyglycol ether of a fatty alcohol containing C12 and (an average) of 9 oxyethylene groups (e.g. Laureth-9), C12 and (an average of) 10 oxyethylene groups (e.g. Genapol X-100), C12 and (an average of) 23 oxyethylene groups (e.g. Brij 35), C16 and (an average of) 10 oxyethylene groups (e.g. Brij C10), C18 and (an average of) 100 oxyethylene groups (e.g. Brij S100), C18 and (an average of) 10 oxyethylene groups (e.g. Eco Brij S10).

In an embodiment, the polyglycol ether of a fatty alcohol comprises Laureth-9. Laureth-9 itself is 3,6,9,12,15,18,21,24,27-nonaoxanonatriacontan-1-ol. In practical sense it is a lauryl alcohol which is etherified with an average of 9 oxyethylene groups, a mixture with a spread from 1-20 oxyethylene groups.

Laureth-9 is a micelle-forming surfactant, meaning it is capable to form micelles in solution. To this end, the so-called critical micelle concentration (CMC) of a surfactant is defined as the concentration of said surfactant above which micelles start to form. At a certain temperature, the so-called cloud point, the surfactant forms insoluble aggregates in the aqueous medium and phase-separates.

In an embodiment, the polyglycol ether of a fatty alcohol is preferably present in the mixture at a concentration of equal or above the CMC. Preferably, the polyglycol ether of a fatty alcohol is present at a concentration of at least 3 times the CMC. More preferably, the polyglycol ether of a fatty alcohol is present at a concentration of at least 5 times the CMC. Even more preferably, the polyglycol ether of a fatty alcohol is present at a concentration of at least 10 or 20 times the CMC. Most preferably, the polyglycol ether of a fatty alcohol is present at a concentration of at least 30 times the CMC. Preferably, the aqueous solution comprises at least 0.001 w/w%, more preferably at least 0.002 w/w%, even more preferably at least 0.004 w/w%, yet even more preferably at least 0.006 w/w%, yet even more preferably at least 0.008 w/w%, yet even more preferably at least 0.01 w/w%, yet even more preferably at least 0.03 w/w%, most preferably at least 0.05 w/w% polyglycol ether of a fatty alcohol, wherein the polyglycol ether of a fatty alcohol is preferably Laureth-9.

In another embodiment, the aqueous solution preferably comprises between 0.001-0.5 w/w%, more preferably between 0.004-0.5 w/w%, even more preferably between 0.004-0.1 w/w%, yet even more preferably between 0.004-0.06 w/w% polyglycol ether of a fatty alcohol, wherein the polyglycol ether of a fatty alcohol is preferably Laureth-9. Even more preferably, the aqueous solution comprises between 0.001-0.05 w/w% polyglycol ether of a fatty alcohol, wherein the polyglycol ether of a fatty alcohol is preferably Laureth-9. Even more preferably, the aqueous solution comprises between 0.002-0.02 w/w%, even more preferably between 0.004-0.01 w/w% polyglycol ether of a fatty alcohol, wherein the polyglycol ether of a fatty alcohol is preferably Laureth-9. The exact concentration of the polyglycol ether of a fatty alcohol can also be adjusted depending on the LPS content in the collagen-containing and/or collagen-derived product. For example, if the starting material has a low LPS content, a low concentration of the polyglycol ether of a fatty alcohol (i.e. not largely exceeding the CMC value) may be sufficient. The concentration of the polyglycol ether of a fatty alcohol may be expressed in relation to the amount of collagen-containing and/or collagen-derived product present in the aqueous solution. Preferably, the polyglycol ether of a fatty alcohol, is present at a concentration between 0.1-10 milligram, even more preferably between 0.2-8 milligram, yet even more preferably between 0.9-8 milligram per 1 gram collagen-containing and/or collagen-derived product present in the aqueous solution. In addition or alternatively, the polyglycol ether of a fatty alcohol is preferably present at a concentration of at least 0.1, 0.2, 0.4, 0.6, 0.8, 1 milligram polyglycol ether of a fatty alcohol per 1 gram collagen-containing and/or collagen-derived product. In addition or alternatively, the polyglycol ether of a fatty alcohol is preferably present at a concentration of at most 10, 8, 6, 5, 4, 2 milligram polyglycol ether of a fatty alcohol per 1 gram collagen-containing and/or collagen-derived product.

In a preferred embodiment, the aqueous solution comprises between 6-10 w/w% collagen-containing and/or collagen-derived product and between 0.004-0.06 w/w% polyglycol ether of a fatty alcohol, wherein the polyglycol ether of a fatty alcohol is preferably Laureth-9. The percentage of CMC can even vary more widely, as it is known that it also depends on the composition of the aqueous solution.

In certain embodiments, for example when the polyglycol ether of a fatty alcohol contains and/or contains on average between 1-8, preferably between 1-5, more preferably between 1-3 oxyethylene groups, an ethanolic solution may be used or a solution comprising another co-solvent as disclosed herein. In addition or alternatively, when the polyglycol ether of a fatty alcohol contains and/or contains on average less than 5, 4, 3, 2, 1 oxyethylene groups, an ethanolic solution may be used or a solution comprising another co-solvent as disclosed herein, which may give similar or even better results than when an aqueous solution is used. The ethanolic solution may for example comprise 10-70 (v/v)%, 20-60 (v/v)% or 30-50% ethanol.

The contacting period between the collagen-containing and/or collagen-derived product and the polyglycol ether of a fatty alcohol is sufficiently long to allow mixing. In addition or alternatively, the contacting period is chosen such that the envisioned endotoxin activity is reached. Preferably, the contacting period between the collagen-containing and/or collagen-derived product and the polyglycol ether of a fatty alcohol is at least 1 minute. More preferably, the contacting period is at least 5 minutes, even more preferably at least 10 minutes, yet even more preferably at least 20 minutes, yet even more preferably at least 30 minutes, yet even more preferably at least 60 minutes. More preferably, the collagen-containing and/or collagen-derived product and the polyglycol ether of a fatty alcohol are incubated between 1-60 minutes, more preferably between 5-30 minutes, most preferably between 20-30 minutes.

In an embodiment, the collagen-containing and/or collagen-derived product may be recovered by contacting the mixture with an adsorbent. The adsorbent can be any suitable adsorbent, capable of binding the surfactant and preferably also LPS. It is of advantage that the aqueous medium is free flowing when adding the surfactant and when brought in contact with the adsorbent. Although the viscosity and gelling temperature varies across different collagen-containing and/or collagen-derived products, solutions are likely to be free flowing and well processable at a temperature of at least 30 °C. A free-flowing solution does not only allow optimal contact between the medium and absorbent, but also enables proper separation of the adsorbent.

In an embodiment, the adsorbent is solid.

In an embodiment, the solid adsorbent is hydrophobic.

In an embodiment, the solid adsorbent comprises activated carbon, for example Norit SX Plus or Norit ROX 0.8 (Cabot, the Netherlands).

In an embodiment, the absorbent is provided in a filter. Alternatively or additionally, the adsorbent can be stacked in a column. The contacting step between the mixture and solid adsorbent is performed for a sufficient amount of time, this to allow proper adsorption of the polyglycol ether of a fatty alcohol and/or LPS.

In an embodiment, the mixture may be recovered by e.g. filtration, sedimentation or centrifugation. Preferably, the mixture is contacted with the solid adsorbent for 5 minutes to an hour, more preferably for 10-30 minutes. A shorter time period is possible, but may require more adsorbent as compared to incubation for longer time periods in order to achieve the desired recovered product. A longer time period is also possible, but less desired in view of process efficiency. Preferably, the adsorption step may be performed once.

In an embodiment, the method disclosed herein may comprise contacting the collagen-containing and/or collagen-derived product comprising lipopolysaccharides with the polyglycol ether of a fatty alcohol, followed by a drying step, thereby allowing to obtain a powder. In an embodiment, the method disclosed herein comprises a step of contacting the mixture with the adsorbent, followed by a step of drying thereby allowing to obtain a powder. Preferably, the drying is spray drying. In an embodiment, the step of drying may be part of the (optional) step of recovering the collagen-containing and/or collagen-derived product. When the step of drying is part of the (optional) step of recovering the collagen-containing and/or collagen-derived product, the (optional) step of contacting the mixture with an adsorbent and (optional) recovery by e.g. filtration, sedimentation or centrifugation if followed by a step of drying, thereby allowing to obtain a powder.

Further described is the use of a polyglycol ether of a fatty alcohol to reduce LPS (activity) in collagen-containing and/or collagen-derived products.

Further described is the use of a polyglycol ether of lauryl alcohol to reduce LPS (activity) in collagen-containing and/or collagen-derived products.

In an aspect, the present invention relates to a collagen-containing and/or collagen-derived product, optionally obtainable by the method disclosed herein. In a preferred embodiment, the collagen-containing and/or collagen-derived product is gelatin or hydrolyzed gelatin. In another preferred embodiment, the collagen-containing and/or collagen-derived product is collagen hydrolysate.

In an embodiment, the collagen-containing and/or collagen-derived product, optionally obtainable by the methods of the preceding claims, preferably has an LPS content of less than 3000 EU/g, preferably less than 1000 EU/g, more preferably less than 100 EU/g, even more preferably less than 10 EU/g, most preferably less than 1 EU/g.

In an embodiment, the collagen-containing and-or collagen-derived product may comprise one or more polyglycol ether of a fatty alcohol as disclosed herein, preferably Laureth-9. In an embodiment the collagen-containing and-or collagen-derived product may comprise less than 1000 ppm residue of the polyglycol ether of a fatty alcohol, optionally even less than 750 ppm, or even less than 500 ppm, or even less than 250 ppm, or even less than 100 ppm, or even less than 75 ppm, or even less than 50 ppm, or even less than 25 ppm, or even less than 10 ppm, or even less than 5 ppm, or even less than 2 ppm, or even less than 1 ppm, or even less than 0.1 ppm. In an embodiment, the amount of polyglycol ether of a fatty alcohol in the collagen-containing and/or collagen-derived product is preferably 0.01 - 10 ppm. In an embodiment, the amount of polyglycol ether of a fatty alcohol in the collagen-containing and/or collagen-derived product is preferably 0.05 - 5 ppm. In an embodiment, the amount of polyglycol ether of a fatty alcohol in the collagen-containing and/or collagen-derived product is preferably 0.1 - 1 ppm. In an embodiment, the amount of polyglycol ether of a fatty alcohol in the collagen-containing and/or collagen-derived product is preferably 0.2 - 0.5 ppm. In an embodiment, the amount of polyglycol ether of a fatty alcohol in the collagen-containing and/or collagen-derived product is preferably 0.3 - 0.4 ppm.

In addition or alternatively, the invention also relates to a collagen-containing and/or collagen-derived product, optionally obtainable by the methods of the preceding claims, comprising an LPS content of less than 3000 EU/g, preferably less than 1000 EU/g, more preferably less than 100 EU/g, even more preferably less than 10 EU/g, most preferably less than 1 EU/g and between 0.001-100, 0.001-50, 0.001-25, 0.001-10, 0.001-5, 0.001-1 ppm residue of the polyglycol ether of a fatty alcohol, preferably between 0.004-2, 0.004-1, 0.004-0.8, 0.004-0.6, 0.004-0.4, 0.004-0.2 ppm residue of the polyglycol ether of a fatty alcohol, wherein the polyglycol ether of a fatty alcohol is preferably Laureth-9.

In an embodiment, the collagen- containing and/or collagen-derived product, optionally obtainable by the methods of the preceding claims, may find applications in a variety of industries, for example in the food, pharmaceutical and cosmetic industry. In view of this the collagen-containing and/or collagen-derived product, optionally obtainable by a method the invention, may be for use in a medical device, pharmaceuticals and/or cosmetic applications Said collagen- containing and/or collagen-derived product may be used as a gelling agent, a texturizer and/or a hydrogel. In view of this, the current invention further relates to the use of a collagen-containing and/or collagen-derived product, preferably the use of a collagen-containing and/or collagen-derived product in a medical device and/or in pharmaceuticals and/or in in a cosmetic (application).

### BRIEF DESCRIPTION OF FIGURES

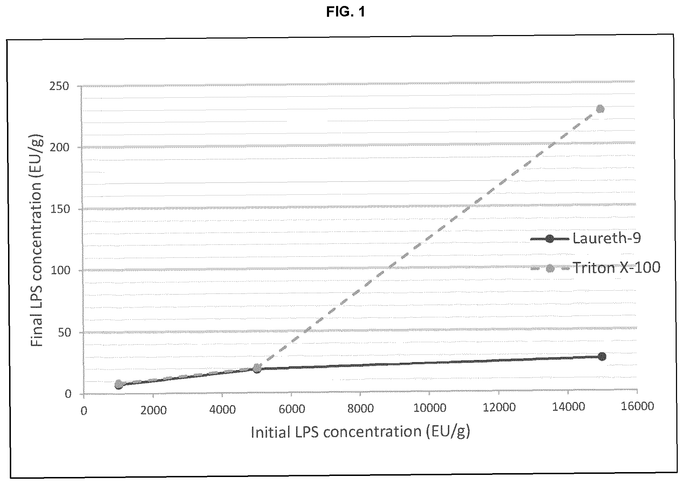

Figure 1 is a graph, demonstrating the effectiveness of Laureth-9 and Triton X-100 to remove LPS from gelatins with different starting levels of LPS. The x-axis shows the LPS content (EU/g) prior to exposure to Laureth-9 or Triton X-100 and the y-axis shows the LPS content (EU/g) after exposure to Laureth-9 or Triton X-100.

### EXAMPLES

### Example 1. The effect of Triton X-100 and Laureth-9 in the reduction of LPS activity.

To determine the effect of Laureth-9 on reducing LPS activity, an 8 w/w% (high bloom, high viscosity, initial LPS content 800-1200 EU/g) porcine-derived gelatin solution was prepared in ultrapure water and exposed to different concentrations of Laureth-9. Laureth-9 was added to the gelatin solution at a concentration of 0.0015 w/w%, 0.004 w/w% or 0.015 w/w% and mixed for 30 minutes. Afterwards, the gelatin solution (comprising Laureth-9) was filtered, recovering the gelatin solution. The unfiltered gelatin solution, along with the first 25 mL and the last 100 mL of the filtered gelatin solution were sampled and stored at -20°C until analysis. To compare the potential effect of Laureth-9 to the prior art, one example using 0.09 w/w% Triton X-100 was included in the experiment.

The LPS concentration in the recovered gelatin samples was determined using the EndoZyme II LPS quantification rFC assay kit (Biomerieux, Marcy-I'Etoile, France), which is a type of LAL assay. Recombinant Factor C (rFC) is activated by endotoxin binding and in a cascade of reaction an active enzyme then cleaves a synthetic substrate, resulting in the generation of a fluorogenic compound. The fluorogenic compound is then measured and the intensity of the compound is directly proportional to the amount of endotoxin present in the analysed sample.

The analysis was performed using a Biotek Synergy MX Microplate Reader (Agilent Technologies, California, United States), measuring the fluorescent signal at excitation wavelength of 380nm and emission wavelength of 445nm..

Table 1 indicates that the use of 0.004 w/w% and 0.015 w/w% Laureth-9 results in lower endotoxin levels than when using Triton X-100. In addition, it is also shown that the additional filtration step is not mandatory, as acceptable LPS levels (i.e. below 10 EU/g) are already reached prior to filtration.

**Table 1. Lipopolysaccharides (LPS) reduction in a porcine-derived gelatin solution after exposure to various concentrations of Laureth-9 and Triton X-100**

| Surfactant name | Surfactant concentration | Filtrated Gelatin Solution Quantity (mL) | LPS concentration (EU/g) | Spike Recovery (%) |
|---|---|---|---|---|
| Triton X-100 | 0.090 w/w% | Unfiltered | **6** | **107** |
| | | 25 | **5** | **119** |
| | | 100 | **8** | **99** |
| | 0.0015 w/w% | Unfiltered | **210** | **112** |
| | | 25 | **214** | **122** |
| | | 100 | **1188** | **126** |
| | 0.004 w/w% | Unfiltered | **2** | **93** |
| Laureth-9 | | 25 | **4** | **103** |
| | | 100 | **3** | **111** |
| | 0.015 w/w% | Unfiltered | **3** | **115** |
| | | 25 | **2** | **77** |
| | | 100 | **2** | **90** |

Similar results were obtained as for the 0.015 w/w% Laureth-9 groups when testing other (higher) concentrations of Laureth-9, for example: 0.01 w/w%, 0.025 w/w% and 0.1 w/w% Laureth-9. In addition, a similar result was obtained when other collagen-containing and/or collagen-derived product were used, for example high bloom high viscosity gelatin, low bloom low viscosity gelatin, and (chemically) modified gelatins with one or more functionalized groups to the gelatin backbone, such as methacryloyl (GeIMA), desaminotyrosine (GeIDAT).

The experiment (also using the high bloom, high viscosity porcine-derived gelatin) was also performed to evaluate the reduction of LPS activity of several polyglycol ethers of a fatty alcohol with variations in fatty alcohol length and average number of oxyethylene groups (Table 2). Table 2 shows that LPS was effectively reduced for the different polyglycol ethers of a fatty alcohol tested.

**Table 2. Lipopolysaccharides (LPS) reduction in a porcine-derived gelatin solution after exposure to various surfactants.**

| Product | Fatty alcohol length | Average number of oxyethylene groups | Surfactant concentration (w/w%) | LPS concentration (EU/g): |
|---|---|---|---|---|
| Genapol X-100 | 12 | 10 | 0.01 | 12 |
| Brij 35 | 12 | 23 | 0.01 | 289 |
| | | | 0.1 | 352 |
| Brij C10 | 16 | 10 | 0.1 | 38 |
| | | | 1 | 28 |
| Brij S100 | 18 | 100 | 0.01 | 366 |
| | | | 0.1 | 307 |
| Eco Brij S10 | 18 | 10 | 0.1 | 31 |
| | | | 1 | 53 |

### Example 2. The effect of Laureth-9 in the reduction of LPS activity in porcine-derived and bovine-derived gelatin.

To determine the effect of Laureth-9 on LPS removal in different types of gelatin, a porcine-derived gelatin (type A) and bovine-derived gelatin (type B), with an initial LPS concentration of 14000-16000 EU/g and 4000-7000 EU/g, respectively, were evaluated. The same protocol was followed as in example 1.

Laureth-9 demonstrated a satisfactory reduction in LPS concentration on both type A and type B gelatin, reaching LPS concentrations of around and below 3000 EU/g (Table 3).

**Table 3. Lipopolysaccharides (LPS) reduction in a porcine and bovine-derived gelatin solution after exposure to various concentrations of Laureth-9.**

| Gelatin Name | Surfactant Concentration | Filtrated Gelatin Solution Quantity (mL) | LPS Concentration (EU/g) | Spike Recovery (%) |
|---|---|---|---|---|
| Gelatin type A | 0.004 w/w% | Unfiltered | **21** | **128** |
| | | 25 | **31** | **165** |
| | | 100 | **20** | **148** |
| | 0.015 w/w% | Unfiltered | **19** | **137** |
| | | 25 | **35** | **178** |
| | | 100 | **22** | **133** |
| Gelatin type B | 0.004 w/w% | Unfiltered | **1797** | **79** |
| | | 25 | **1958** | **94** |
| | | 100 | **2342** | **88** |
| | 0.015 w/w% | Unfiltered | **30** | **97** |
| | | 25 | **19** | **90** |
| | | 100 | **17** | **82** |

### Example 3. Effectiveness of LPS removal by Laureth-9 and Triton X-100

To compare the effectiveness of Laureth-9 and Triton X-100, gelatins with different starting concentrations of LPS (i.e. 1000 EU/g, 5000 EU/g and 15000 EU/g) were incubated with either Laureth-9 or Triton X-100. After incubation, the recovered gelatin samples were tested for their LPS content, according to as described in Example 1.

As can be seen in Figure 1, when working with gelatin with relatively high LPS activity (i.e. > 5000 EU/g) the LPS reduction efficacy of Laureth-9 higher than that of Triton X-100.

## Claims

1. Method for reducing lipopolysaccharides activity in a collagen-containing and/or collagen-derived product, said method comprising the steps of:
- providing a collagen-containing and/or collagen-derived product comprising lipopolysaccharides,
- contacting the collagen-containing and/or collagen-derived product comprising lipopolysaccharides with a polyglycol ether of a fatty alcohol to provide a mixture,
- recovering a collagen-containing and/or collagen-derived product, wherein the lipopolysaccharides activity is preferably less than 3000 EU/g, more preferably less than 1000 EU/g, even more preferably less than 100 EU/g, yet even more preferably less than 10 EU/g, most preferably less than 1 EU/g.

2. Method according to claim 1, wherein the collagen-containing and/or collagen-derived product is selected from the group consisting of extracellular matrix, native collagen, gelatin, (chemically) modified gelatin, gelatin hydrolysate, and mixtures thereof.

3. Method according to any of the preceding claims, wherein the collagen-containing and/or collagen-derived product is in the form of an aqueous solution, preferably comprising between 1-50 w/w% collagen-containing and/or collagen-derived product.

4. Method according to any of the preceding claims, wherein the polyglycol ether of a fatty alcohol is a polyglycol ether of lauryl alcohol and/or contains 1-20 oxyethylene groups.

5. Method according to any of the preceding claims, wherein the polyglycol ether of a fatty alcohol comprises Laureth-9.

6. Method according to any of claims 3-5, wherein the aqueous solution comprises at least 0.001 w/w%, preferably between 0.001-0.05 w/w%, of the polyglycol ether of a fatty alcohol.

7. Method according to any of the preceding claims, wherein the mixture is contacted with an adsorbent.

8. Method according to any of the preceding claims, wherein the collagen-containing and/or collagen-derived product is recovered by filtration.

9. Use of a polyglycol ether of a fatty alcohol to reduce lipopolysaccharides activity in collagen-containing and/or collagen-derived products, wherein the polyglycol ether of a fatty alcohol is preferably a polyglycol ether of lauryl alcohol.

10. Collagen-containing and/or collagen-derived product, obtainable by the method of any one of claims 1-8.

11. Collagen-containing and/or collagen-derived product according to claim 10, having a lipopolysaccharides content of less than 3000 EU/g.

12. Collagen-containing and/or collagen-derived product according to claim 10 or 11, comprising a polyglycol ether of a fatty alcohol in an amount of less than 10 ppm, preferably less than 2 ppm, more preferably less than 1 ppm, even more preferably less than 0.1 ppm.

13. Collagen-containing and/or collagen-derived product according to any one of claims 10-12, comprising a polyglycol ether of a fatty alcohol, preferably Laureth-9.

14. Collagen-containing and/or collagen-derived product according to any one of claims 10-13, wherein the collagen-containing and/or collagen-derived product is one or more selected from the group consisting of gelatin, hydrolysed gelatin and collagen hydrolysate.

15. Use of the collagen-containing and/or collagen-derived product according to any one of claims 10-14 in a medical device, pharmaceuticals and/or cosmetic applications.

## Patentansprüche

1. Verfahren zur Verringerung der Aktivität von Lipopolysacchariden in einem kollagenhaltigen und/oder kollagenabgeleiteten Produkt, wobei das Verfahren die folgenden Schritte umfasst:
- Bereitstellen eines kollagenhaltigen und/oder kollagenabgeleiteten Produkts, das Lipopolysaccharide umfasst,
- In-Kontakt-Bringen des Lipopolysaccharide umfassenden kollagenhaltigen und/oder kollagenabgeleiteten Produkts mit einem Polyglykolether eines Fettalkohols, so dass ein Gemisch bereitgestellt wird,
- Gewinnen eines kollagenhaltigen und/oder kollagenabgeleiteten Produkts, wobei die Aktivität der Lipopolysaccharide bevorzugt weniger als 3000 EU/g, bevorzugter weniger als 1000 EU/g, noch bevorzugter weniger als 100 EU/g, noch weiter bevorzugt weniger als 10 EU/g, am meisten bevorzugt weniger als 1 EU/g beträgt.

2. Verfahren nach Anspruch 1, wobei das kollagenhaltige und/oder kollagenabgeleitete Produkt aus der Gruppe bestehend aus extrazellulärer Matrix, nativem Kollagen, Gelatine, (chemisch) modifizierter Gelatine, Gelatinehydrolysat und Gemischen davon ausgewählt ist.

3. Verfahren nach einem der vorhergehenden Ansprüche, wobei das kollagenhaltige und/oder kollagenabgeleitete Produkt in Form einer wässrigen Lösung vorliegt, die vorzugsweise zwischen 1-50 Gew.-% kollagenhaltiges und/oder kollagenabgeleitetes Produkt umfasst.

4. Verfahren nach einem der vorhergehenden Ansprüche, wobei der Polyglykolether eines Fettalkohols ein Polyglykolether von Laurylalkohol ist und/oder 1-20 Oxyethylengruppen enthält.

5. Verfahren nach einem der vorhergehenden Ansprüche, wobei der Polyglykolether eines Fettalkohols Laureth-9 umfasst.

6. Verfahren nach einem der Ansprüche 3-5, wobei die wässrige Lösung mindestens 0,001 Gew.-%, bevorzugt zwischen 0,001-0,05 Gew.-%, des Polyglykolethers eines Fettalkohols umfasst.

7. Verfahren nach einem der vorhergehenden Ansprüche, wobei das Gemisch mit einem Adsorptionsmittel in Kontakt gebracht wird.

8. Verfahren nach einem der vorhergehenden Ansprüche, wobei das kollagenhaltige und/oder kollagenabgeleitete Produkt durch Filtration gewonnen wird.

9. Verwendung eines Polyglykolethers eines Fettalkohols zur Verringerung der Aktivität von Lipopolysacchariden in kollagenhaltigen und/oder kollagenabgeleiteten Produkten, wobei es sich bei dem Polyglykolether eines Fettalkohols vorzugsweise um einen Polyglykolether von Laurylalkohol handelt.

10. Kollagenhaltiges und/oder kollagenabgeleitetes Produkt, erhältlich mit dem Verfahren gemäß einem der Ansprüche 1-8.

11. Kollagenhaltiges und/oder kollagenabgeleitetes Produkt nach Anspruch 10, aufweisend einen Gehalt an Lipopolysacchariden von weniger als 3000 EU/g.

12. Kollagenhaltiges und/oder kollagenabgeleitetes Produkt nach Anspruch 10 oder 11, umfassend einen Polyglykolether eines Fettalkohols in einer Menge von weniger als 10 ppm, bevorzugt weniger als 2 ppm, bevorzugter weniger als 1 ppm, noch bevorzugter weniger als 0,1 ppm.

13. Kollagenhaltiges und/oder kollagenabgeleitetes Produkt nach einem der Ansprüche 10-12, umfassend einen Polyglykolether eines Fettalkohols, vorzugsweise Laureth-9.

14. Kollagenhaltiges und/oder kollagenabgeleitetes Produkt nach einem der Ansprüche 10-13, wobei es sich bei dem kollagenhaltigen und/oder kollagenabgeleiteten Produkt um ein oder mehrere aus der Gruppe bestehend aus Gelatine, hydrolysierter Gelatine und Kollagenhydrolysat ausgewählte Produkte handelt.

15. Verwendung des kollagenhaltigen und/oder kollagenabgeleiteten Produkts nach einem der Ansprüche 10-14 in einer medizinischen Vorrichtung, Pharmazeutika und/oder kosmetischen Anwendungen.

## Revendications

1. Procédé pour réduire l'activité de lipopolysaccharides dans un produit contenant un collagène et/ou dérivé d'un collagène, ledit procédé comprenant les étapes de :
- fourniture d'un produit contenant un collagène et/ou dérivé d'un collagène comprenant des lipopolysaccharides,
- mise en contact du produit contenant un collagène et/ou dérivé d'un collagène comprenant des lipopolysaccharides avec un éther polyglycolique d'un alcool gras pour fournir un mélange,
- récupération d'un produit contenant un collagène et/ou dérivé d'un collagène, dans lequel l'activité de lipopolysaccharides est de préférence inférieure à 3 000 UE/g, plus préférablement inférieure à 1 000 UE/g, encore plus préférablement inférieure à 100 UE/g, même encore plus préférablement inférieure à 10 UE/g, le plus préférablement inférieure à 1 UE/g.

2. Procédé selon la revendication 1, dans lequel le produit contenant un collagène et/ou dérivé d'un collagène est choisi dans le groupe constitué par une matrice extracellulaire, un collagène natif, une gélatine, une gélatine (chimiquement) modifiée, un hydrolysat de gélatine, et leurs mélanges.

3. Procédé selon l'une quelconque des revendications précédentes, dans lequel le produit contenant un collagène et/ou dérivé d'un collagène est sous la forme d'une solution aqueuse, comprenant de préférence entre 1-50 % p/p de produit contenant un collagène et/ou dérivé d'un collagène.

4. Procédé selon l'une quelconque des revendications précédentes, dans lequel l'éther polyglycolique d'un alcool gras est un éther polyglycolique d'alcool laurylique et/ou contient 1-20 groupes oxyéthylène.

5. Procédé selon l'une quelconque des revendications précédentes, dans lequel l'éther polyglycolique d'un alcool gras comprend le Laureth-9.

6. Procédé selon l'une quelconque des revendications 3 à 5, dans lequel la solution aqueuse comprend au moins 0,001 % p/p, de préférence entre 0,001 et 0,05 % p/p, de l'éther polyglycolique d'un alcool gras.

7. Procédé selon l'une quelconque des revendications précédentes, dans lequel le mélange est mis en contact avec un adsorbant.

8. Procédé selon l'une quelconque des revendications précédentes, dans lequel le produit contenant un collagène et/ou dérivé d'un collagène est récupéré par filtration.

9. Utilisation d'un éther polyglycolique d'un alcool gras pour réduire l'activité de lipopolysaccharides dans des produits contenant un collagène et/ou dérivés d'un collagène, dans laquelle l'éther polyglycolique d'un alcool gras est de préférence un éther polyglycolique d'alcool laurylique.

10. Produit contenant un collagène et/ou dérivé d'un collagène, pouvant être obtenu par le procédé selon l'une quelconque des revendications 1 à 8.

11. Produit contenant un collagène et/ou dérivé d'un collagène selon la revendication 10, ayant une teneur en lipopolysaccharides inférieure à 3 000 UE/g.

12. Produit contenant un collagène et/ou dérivé d'un collagène selon la revendication 10 ou 11, comprenant un éther polyglycolique d'un alcool gras en une quantité inférieure à 10 ppm, de préférence inférieure à 2 ppm, plus préférablement inférieure à 1 ppm, encore plus préférablement inférieure à 0,1 ppm.

13. Produit contenant un collagène et/ou dérivé d'un collagène selon l'une quelconque des revendications 10 à 12, comprenant un éther polyglycolique d'un alcool gras, de préférence le Laureth-9.

14. Produit contenant un collagène et/ou dérivé d'un collagène selon l'une quelconque des revendications 10 à 13, dans lequel le produit contenant un collagène et/ou dérivé d'un collagène est un ou plusieurs choisis dans le groupe constitué par une gélatine, une gélatine hydrolysée et un hydrolysat de collagène.

15. Utilisation du produit contenant un collagène et/ou dérivé d'un collagène selon l'une quelconque des revendications 10 à 14 dans un dispositif médical, des applications pharmaceutiques et/ou cosmétiques.
